# EUROPEAN PATENT SPECIFICATION

(11) **EP 3 067 043 B1**
(45) Date of publication and mention of the grant of the patent: **30.11.2022**
(21) Application number: 16157767.1
(22) Date of filing: 04.11.2008
(51) Int. Cl.: A61K 9/20, A61P 43/00, A61P 13/12, A61P 9/10, A61P 9/04, A61P 9/00, A61K 31/41, A61K 31/216

(54) **PHARMACEUTICAL COMPOSITIONS BASED ON SUPERSTRUCTURES OF ANGIOTENSIN RECEPTOR ANTAGONIST/BLOCKER (ARB) AND NEUTRAL ENDOPEPTIDASE (NEP) INHIBITOR**
PHARMAZEUTISCHE ZUSAMMENSETZUNGEN AUF BASIS VON SUPERSTRUKTUREN AUS EINEM ANGIOTENSINREZEPTORANTAGONISTEN/-BLOCKER (ARB) UND EINEM NEUTRALENDOPEPTIDASEHEMMER (NEP)
COMPOSITIONS PHARMACEUTIQUES BASÉES SUR DES SUPERSTRUCTURES D'ANTAGONISTE/BLOQUEUR DU RÉCEPTEUR DE L'ANGIOTENSINE (ARB) ET DE L'INHIBITEUR DE L'ENDOPEPTIDASE NEUTRE (NEP)

(30) Priority: 06.11.2007 US 985668 P
(43) Date of publication of application: 14.09.2016
(62) Divisional of application: 10173506.6
(73) Proprietor: Novartis AG, 4056 Basel (CH)
(72) Inventor: Al-Fayoumi, Suliman, Morristown, New Jersey 07960 (US); Hu, Jiahui, Bridgewater, New Jersey 08807 (US); Kumaraperumal, Natrajan, Wayne, 07470 (US); Royce, Alan Edward, Saylorsburg, PA 18353 (US); Ruegger, Colleen, Morris Plains, NJ 07950 (US); Zannou, Erika Aina, Edison, New Jersey 08820 (US)
(74) Representative: Larbig, Karen Dorothee

(56) References cited:
- WO-A-00/38676
- WO-A-2005/014043
- WO-A-2007/056546
- US-A1- 2002 098 241
- US-B1- 6 248 729
- AULAKH ET AL: "An update on non-peptide angiotensin receptor antagonists and related RAAS modulators", LIFE SCIENCES, PERGAMON PRESS, OXFORD, GB, vol. 81, no. 8, 2 August 2007 (2007-08-02) , pages 615-639, XP022207851, ISSN: 0024-3205

## Description

### Field of the Invention

The present invention relates to a solid oral dosage form comprising the therapeutic agent LCZ696. Such a pharmaceutical composition may be prepared by a dry formulation process such as direct compression or compaction process to form a solid oral dosage form.

### Background of the Invention

Angiotensin II is a hormone that causes blood vessels to constrict which can result in hypertension and strain on the heart. This hormone interacts with specific receptors on the surface of target cells. Two receptor subtypes for angiotensin II, e.g., AT1 and AT2, have been identified thus far. In recent times, great effort have been made to identify substances that bind to the AT1 receptor. Angiotensin receptor blockers (ARBs, angiotensin II antagonists) prevent angiotensin II from binding to its receptors in the walls of blood vessels thereby reducing blood pressure. Because of the inhibition of the AT1 receptor, such antagonists can be used, therefore, as anti-hypertensives or for the treatment of congestive heart failure, among other indications.

Neutral endopeptidase (EC 3.4.24.11; enkephalinase; atriopeptidase; NEP) is a zinc-containing metalloprotease that cleaves a variety of peptide substrates on the amino side of hydrophobic residues [see Pharmacol Rev, Vol. 45, p. 87 (1993)]. Substrates for this enzyme include, but are not limited to, atrial natriuretic peptide (ANP, also known as ANF), brain natriuretic peptide (BNP), met- and leu-enkephalin, bradykinin, neurokinin A, endothelin-1 and substance P. ANP is a potent vasorelaxant and natriuretic agent [see J Hypertens, Vol. 19, p. 1923 (2001)]. Infusion of ANP in normal subjects resulted in a reproducible, marked enhancement of natriuresis and diuresis, including increases in fractional excretion of sodium, urinary flow rate and glomerular filtration rate [see J Clin Pharmacol, Vol. 27, p. 927 (1987)]. However, ANP has a short half-life in circulation, and NEP in kidney cortex membranes has been shown to be the major enzyme responsible for degrading this peptide [see Peptides, Vol. 9, p. 173 (1988)]. Thus, inhibitors of NEP (neutral endopeptidase inhibitors, NEPi) should increase plasma levels of ANP and, hence, are expected to induce natriuretic and diuretic effects.

While substances, such as angiotensin receptor blockers and neutral endopeptidase inhibitors may be useful in the control of hypertension, essential hypertension is a polygenic disease and is not always controlled adequately by monotherapy. Approximately 333 million adults in economically developed countries and about 65 million Americans (1 in 3 adults) had high blood pressure in 2000 [see Lancet, Vol. 365, p. 217 (2005); and Hypertension, Vol. 44, p. 398 (2004)]. Prolonged and uncontrolled hypertensive vascular disease ultimately leads to a variety of pathological changes in target organs, such as the heart and kidney. Sustained hypertension can lead as well to an increased occurrence of stroke.

A dual-acting compound or combination, in particular a supramolecular complex of two active agents with different mechanisms of action, or linked pro-drug or in particular a supramolecular complex of two active agents with different mechanisms of action, namely an angiotensin receptor antagonist and a neutral endopeptidase inhibitor has been disclosed in U.S. Patent Applications Nos. 60/735,093 filed November 9, 2005; 60/735,541 filed November 10, 2005; 60/789,332 filed April 4, 2006; and 60/822,086 filed August 11, 2006 and in International publication no. WO 2007/056546. Such supramolecular complexes may be used for the treatment of patients with various cardiovascular and/or renal diseases.

A particularly useful therapeutic agent disclosed in WO 2007/056546 is the supramolecular complex, trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate]hemipentahydrate also referred to as LCZ696.

There is a need to formulate such a supramolecular complex into pharmaceutical compositions, especially solid oral dosage forms, such that the therapeutic benefits of the compounds may be delivered to a patient in need thereof. An object of the present invention is to provide an exemplary solid oral dosage form that may be ingested by a patient.

There has been widespread use of tablets since the latter part of the nineteenth century, and the majority of solid oral dosage forms are marketed as tablets. Major reasons
of tablet popularity as a dosage form are simplicity, low cost, and the speed of production. Other reasons include stability of the drug product, convenience in packaging, shipping and dispensing. To the patient, tablets offer ease of administration, ease of accurate dosage, compactness, portability, and blandness of taste. Thus, it is another object of the present invention to provide a tablet formulation of the therapeutic agent.

The formulation of dual acting compounds such as supramolecular complexes is not trivial since typical formulation techniques may have a negative effect on the drug substance leading to e.g. increased amorphism and/or dissociation of the components of the dual acting compound. In general, one should avoid to expose the therapeutic agent during the formulation to moisture, excessive heat and/or high shear forces. This may pose a number of formulation issues and difficulties which need to be addressed.

### Summary of the Invention

The present invention features solid oral dosage forms for pharmaceutical compounds containing a therapeutic agent, especially a supramolecular complex. In one of aspect of the present invention, the featured supramolecular complex is a dual acting compound. A dual-acting compound or combination features a supramolecular complex of two active agents with different mechanisms of action, or linked pro-drug or in particular a supramolecular complex of two active agents with different mechanisms of action, namely an angiotensin receptor antagonist and a neutral endopeptidase inhibitor. In another aspect of the present invention the featured supramolecular complex is trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate]hemipentahydrate. It was found that with such a formulation, a very different release profile was found than with the two components valsartan and N-(3-carboxy-1-oxopropyl)-(4S)-p-phenylphenylmethyl)-4-amino-2R-methylbutanoic acid ethyl ester alone. In particular, the formulation offers a better exposure and thus bioavailability than valsartan. These unexpected advantages offer the possibility to prepare pharmaceutical compositions with new and lower doses of the therapeutic agent. The pharmaceutical formulations containing a therapeutic agent, especially a supramolecular complex may be manufactured by a dry formulation process such as a direct compression or roller compaction process. Thus, another aspect of the present invention is a solid oral dosage form manufactured by mixing the therapeutic agent with at least one pharmaceutically acceptable excipient, and subsequently directly compressing the mixture with suitable equipment, such as a tablet press, or compacting the mixture with a suitable equipment, such as a roller compactor.

### Brief Description of the Drawings

The accompanying drawing, which is incorporated in and constitute a part of the specification, illustrates an exemplary embodiment of the present invention.

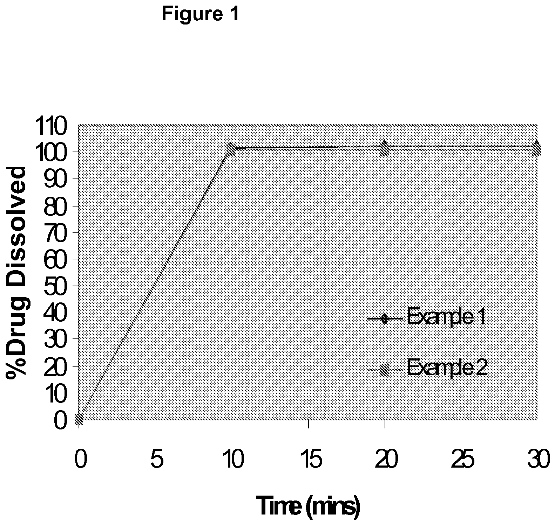

FIG. 1 shows a chart depicting the in vitro dissolution profiles of 5 mg and 50 mg directly compressed tablets of a supramolecular complex trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate]hemipentahydrate.

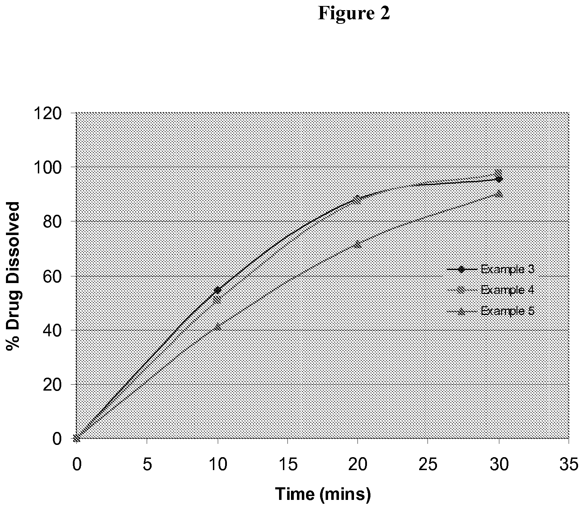

FIG. 2 shows a chart depicting the in vitro dissolution profiles of 100, 200 and 400 mg roller compacted coated tablets of a supramolecular complex trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate]hemipentahydrate at pH 6.8.

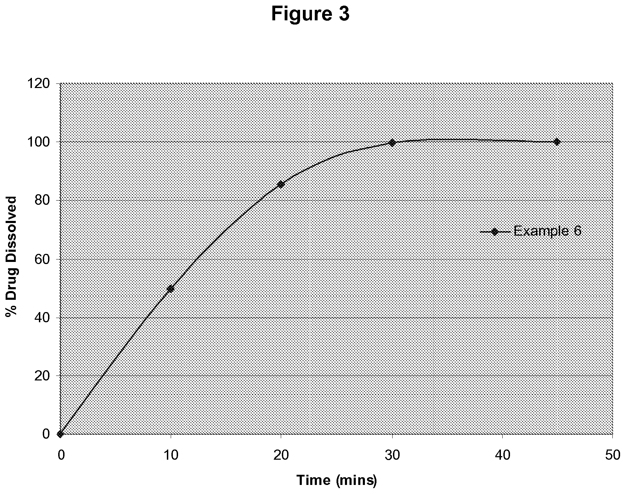

FIG. 3 shows a chart depicting the in vitro dissolution profile of 400 mg roller compacted coated tablets of a supramolecular complex trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate]hemipentahydrate at pH 4.5.

### Detailed Description of the Invention

The present invention relates pharmaceutical compositions comprising a therapeutic agent as set out in the claims. The pharmaceutical compositions of the present invention can be manufactured by a direct compression or preferably a roller compaction process resulting in pharmaceutically acceptable tablets.

As used herein, the term "therapeutic agent" refers to the supramolecular complex, trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate]hemipentahydrate as shown in the following simplified representation:

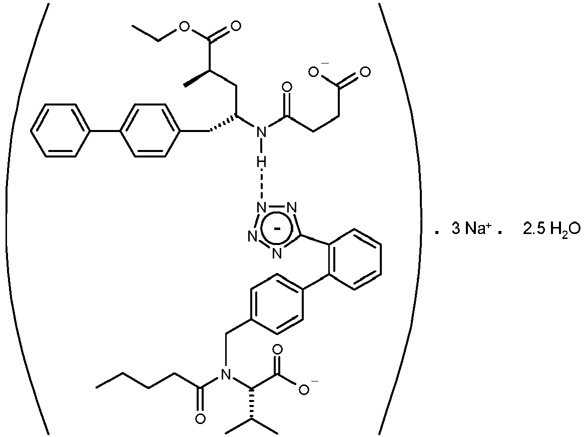

The above therapeutic agent comprises an angiotensin receptor antagonist, a neutral endopeptidase inhibitor (NEPi) and a cation, i.e., Na. This therapeutic agent is fully described with regard to its preparation and its characteristics in WO 2007/056546. This therapeutic agent is a "dual-acting compound" which is intended to describe a compound having two different modes of action simultaneously, i.e., one is the angiotensin receptor blockade resulting from the ARB molecular moiety of the compound, and the other is the neutral endopeptidase inhibition resulting from the NEPi molecular moiety of the compound. The therapeutic agent may be present in the pharmaceutical composition in a range from about 4% to about 60% by weight of the composition.

As used herein the term, "supramolecular complex" is meant to describe an interaction between two pharmaceutically active agents, the cations and any other entity present such as a solvent, in particular water, by means of noncovalent, intermolecular bonding between them. This interaction leads to an association of the species present in the supramolecular complex distinguishing this complex over a physical mixture of species.

As used herein the term "pharmaceutical composition" means, for example, a mixture containing a therapeutically effective amount of a therapeutic compound in a pharmaceutically acceptable carrier to be administered to a mammal, e.g., a human in order to treat kinase dependent diseases. A particularly useful pharmaceutical composition resulting from the present invention is a pharmaceutically acceptable tablet.

As used herein the term "pharmaceutically acceptable" refers to those compounds, materials, compositions and/or dosage forms, which are, within the scope of sound medical judgment, suitable for contact with the tissues of mammals, especially humans, without excessive toxicity, irritation, allergic response and other problem complications commensurate with a reasonable benefit/risk ratio. With respect to a pharmaceutically acceptable tablet, the term also encompasses an acceptable *in vitro* dissolution profile.

The concentration of therapeutic agent in the pharmaceutical composition is present in a therapeutically effective amount which will depend on absorption, inactivation and excretion rates of the therapeutic agent as well as other factors known to one of ordinary skill in the art. Furthermore, it is to be noted that dosage values will also vary with the severity of the condition to be alleviated. It is to be further understood that for any particular recipient, specific dosage regimens should be adjusted over time according to the individual need and the professional judgment of the person administering or supervising the administration of the pharmaceutical compositions. The therapeutic compound may be administered once, or may be divided into a number of smaller doses to be administered at varying intervals of time. Thus, an appropriate therapeutically effective amount is known to one of ordinary skill in the art.

For example, the unit dose of the therapeutic agent will be in the range from about 1 to about 1000, such as 40 to 400 mg (e.g., 100 mg, 200 mg, 400) mg per day. Alternatively lower doses may be given, for example doses of 0.5 to 100 mg; 0.5 to 50 mg; or 0.5 to 20 mg per day. In the present case, it was unexpectantly found that the valsartan component when delivered in the form of the dual acting compound such as the supramolecular complex has a greater exposure and thus higher bioavailability than valsartan on its own. Therefore it is possible to lower the dose with regard to the valsartan component. Specifically, typical doses of valsartan of the Diovan^{®} formulation are 80 mg, 160 mg, and 320 mg. Given that the dual acting compound such as the supramolecular complex comprises the components valsartan and N-(3-carboxy-1-oxopropyl)-(4S)-p-phenylphenylmethyl)-4-amino-2R-methylbutanoic acid ethyl ester, both having very similar molecular weight, in a 1:1 ratio, one would have not foreseen that a 100 mg, 200 mg and 400 mg dose of the dual acting compound would correspond to a 80 mg, 160 mg and 320 mg of valsartan single dose of the Diovan^{®} formulation, respectively, based on the exposure.

As used herein the term "immediate-release" refers to the rapid release of the majority of the therapeutic compound, e.g., greater than about 50%, about 55%, about 60%, about 65%, about 70%, about 75%, about 80%, or about 90% within a relatively short time, e.g., within 1 hour, 40 minutes, 30 minutes or 20 minutes after oral ingestion. Particularly useful conditions for immediate-release are release of at least or equal to about 80% of the therapeutic compound within thirty minutes after oral ingestion. The particular immediate-release conditions for a specific therapeutic compound will be recognized or known by one of ordinary skill in the art. The immediate release profile can be determined from an *in vitro* dissolution test.

The term "dissolution" as used herein refers to a process by which a solid substance, here the active ingredients, is dispersed in molecular form in a medium. The dissolution rate of the active ingredients of the pharmaceutical oral fixed dose combination of the invention is defined by the amount of drug substance that goes in solution per unit time under standardized conditions of liquid/solid interface, temperature and solvent composition. The dissolution rate is measured by standard methods known to the person skilled in the art, see the harmonized procedure set forth in the pharmacopeias USP <711> and EP 2.9.3 and JP. For the purposes of this invention, the test is for measuring the dissolution of the individual active ingredients is performed following pharmacopeia USP <711> at pH 6.8 using a paddle stirring element at 50 rpm (rotations per minute) or alternatively at pH 4.5 using a paddle stirring element at 75 rpm as specified. The dissolution medium is preferably a buffer, typically a phosphate buffer, especially one as described in the example "Dissolution Test".

Accordingly, the present invention provides solid oral dosage forms delivering a therapeutically effective amount of valsartan free acid, or a pharmaceutically acceptable salt thereof, preferably wherein the oral dosage form exhibits an *in vitro* dissolution profile, when measured by the USP paddle method at about 50 rpm in 900 mL of 0.05M at pH 6.8 phosphate buffer and at 37±0.5°C, such that after 10 min, from a mean of about 10% to a mean of about 100% (by weight) of valsartan free acid, or a pharmaceutically acceptable salt thereof, is released, after 20 min, from a mean of about 30% to a mean of about 100% (by weight) of valsartan free acid, or a pharmaceutically acceptable salt thereof, is released, after 30 min, from a mean of about 40% to a mean of about 100% (by weight) of valsartan free acid, or a pharmaceutically acceptable salt thereof, is released.

Moreover, the present invention provides solid oral dosage forms delivering a therapeutically effective amount of valsartan free acid, or a pharmaceutically acceptable salt thereof, preferably wherein the oral dosage form exhibits an *in vitro* dissolution profile, when measured by the USP paddle method at about 75 rpm in 1000 mL of pH 4.5 phosphate buffer and at 37±0.5°C, such that after 10 min, from a mean of about 20% to a mean of about 100% (by weight) of valsartan free acid, or a pharmaceutically acceptable salt thereof, is released, after 20 min, from a mean of about 30% to a mean of about 100% (by weight) of valsartan free acid, or a pharmaceutically acceptable salt thereof, is released, after 30 min, from a mean of about 40% to a mean of about 100% (by weight) of valsartan free acid, or a pharmaceutically acceptable salt thereof, is released.

In one embodiment of the present invention, the therapeutic agent is present in an amount of about 100 mg per unit dosage form, and the oral dosage form exhibits an *in vitro* dissolution profile such that after 10 min, a mean of about 50% of valsartan free acid, is released, after 20 min, a mean of about 85% of valsartan free acid, is released, after 30 min, a mean of about 95% of valsartan free acid, is released. In another embodiment, a the therapeutic agent is present in an amount of about 200 mg per unit dosage form, and the oral dosage form exhibits an *in vitro* dissolution profile such that after 10 min, a mean of about 50% of valsartan free acid, is released, after 20 min, a mean of about 85% of valsartan free acid, is released, after 30 min, a mean of about 95% of valsartan free acid, is released. Yet in another embodiment, a the therapeutic agent is present in an amount of about 400 mg per unit dosage form, and the oral dosage form exhibits an *in vitro* dissolution profile such that after 10 min, a mean of about 40% of valsartan free acid, is released, after 20 min, a mean of about 70% of valsartan free acid, is released, after 30 min, a mean of about 90% of valsartan free acid, is released.

The exact dose of the therapeutic agent and the particular formulation to be administered depend on a number of factors, e.g., the condition to be treated, the desired duration of the treatment and the rate of release of the active agent. For example, the amount of the active agent required and the release rate thereof may be determined on the basis of known *in vitro* or *in vivo* techniques, determining how long a particular active agent concentration in the blood plasma remains at an acceptable level for a therapeutic effect.

Surprisingly, the it was found that the PK/PD profile as obtained with the solid oral dosage form in accordance with the present invention concerning valsartan free acid is very distinct from the single formulation containing only valsartan, in particular the Diovan^{®} formulation. This very distinct and unique pharmacokinetic and pharmacodynamic profile of valsartan free acid following administration of the therapeutic showed a substantially enhanced oral bioavailability (-1.4 to1.6 fold, in particular 1.6 fold) and a trend towards a faster onset (tₘₐₓ 1.8±0.3 h) than that following valsartan administration in the form of the Diovan^{®} formulation (approx. tₘₐₓ 2.6 h). Together, this pharmacokinetic and pharmacodynamic profile of the present dosage form of the therapeutic agent support the further development for the improved treatment of cardiovascular diseases.

Accordingly, the present invention provides solid oral dosage forms delivering a therapeutically effective amount of valsartan free acid, or a pharmaceutically acceptable salt thereof, and a carrier medium, wherein the oral dosage form provides a rapid rate of absorption of valsartan free acid with a tₘₐₓ of 1 to 2.2 h following administration of a single dose of said dosage form. More specifically, a tₘₐₓ of 1.4 to 2.0 h can be observed. This is in stark contrast to the rate of absorption of valsartan following administration of Diovan^{®} where a tₘₐₓ of 2.5 to 4.0 h, more specifically, 2.8 to 3.0 h, is observed. For example, the present invention provides a solid oral dosage form comprising about 200 mg trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate]hemipentahydrate, or a respective amount of valsartan free acid, and a carrier medium, said dosage form providing a tₘₐₓ valsartan free acid of 1.5 to 1.9 h following administration of a single dose of said dosage form.

Furthermore, the present invention provides a solid oral dosage form delivering a therapeutically effective amount of valsartan free acid, or a pharmaceutically acceptable salt thereof, and a carrier medium, wherein the oral dosage form provides a dose-normalized mean plasma exposure (AUC₀₋₂₄) of 230 to 400 ng•h/mL/mg-equivalent following administration of a single dose of said dosage form. More specifically, a dose-normalized geometric mean exposure AUC₀₋₂₄ of 270 to 320 ng•h/mL/mg-equivalent can be observed. The corresponding exposure observed with valsartan administration in the form of Diovan^{®} is much lower. Consequently, the present invention provides a solid oral dosage form delivering a therapeutically effective amount of valsartan free acid, or a pharmaceutically acceptable salt thereof, and a carrier medium, with a mean relative bioavailability of 140 to 185%, such as 150 to 165%, as compared to valsartan following administration of Diovan^{®}. For example, the present invention provides a solid oral dosage form comprising about 200 mg trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate]hemipentahydrate, or a respective amount of valsartan free acid, and a carrier medium, said dosage form providing mean plasma exposure (AUC₀₋₂₄) of 16,000 to 18,000, such as 16,970, ng•h/mL following administration of a single dose of said dosage form.

Thus, with the solid oral dosage form of the present invention, one can achieve not only a faster rate of absorption but also a greater extent of absorption than with Diovan^{®}. These pharmacokinetic properties are expected to lead to therapeutic advantages over valsartan administration on its own.

The solid oral dosage form mentioned above comprises trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate]hemipentahydrate in which both moieties of valsartan or a salt thereof, and (2R,4S)-5-biphenyl-4-yl-4-(3-carboxy-propionylamino)-2-methyl-pentanoic acid ethyl ester or a salt thereof are present in one dual acting molecule or supramolecular complex.

As used herein the term "excipient" refers to a pharmaceutically acceptable inert ingredient that is used in the manufacture of solid oral dosage forms. Examples of categories of excipients include, but are not limited to, binders, disintegrants, lubricants, glidants, stabilizers, fillers and diluents. Excipients can enhance the processing characteristics of the pharmaceutical formulation, i.e., render the formulation more suitable for direct compression by increasing flowability and/or cohesiveness.

As used herein, the term "direct compression" refers to the general process of directly compressing the ingredients in the pharmaceutical formulation (i.e., therapeutic agent and excipients) without changing the physical and chemical properties of the therapeutic agent. The therapeutic agent, along with pharmaceutically acceptable excipients, in the form of powders, are blended in a low shear apparatus, for example a twin shell blender. The blended composition is then filled into a die and directly compressed into a by a punch. A tablet press, for example, can accomplish this compression step. Useful excipients in a direct compression process include, but are not limited to fillers, binders, lubricants and glidants. Direct compression is particularly suitable for a solid oral dosage form having a strength of from 0.5 mg to 200 mg of the therapeutic agent.

As used herein, the term "compaction" refers to the general process of dry granulating to form a tablet (e.g., by slugging or roller compaction). The therapeutic agents and pharmaceutically acceptable excipients are made into slugs (as in slugging) or ribbons (as in roller compaction). The roller compaction process densifies the powder by removing
any air. The densified material is then reduced to a uniform granule size and subsequently compressed. Useful excipients in a roller compaction process include, but are not limited to fillers, binders, lubricants, disintegrants and glidants. Roller compaction is particularly suitable for a solid oral dosage form having a strength of from 50 to 800 mg of the therapeutic agent.

It has been found that roller compaction offers particular advantages for higher doses to provide the therapeutic agent in a suitable tablet size without compromising on the quality of the drug substance. Especially, excessive amorphism as well as separation of the components of the dual acting compound can be minimized or prevented.

A solid oral dosage form according to the invention comprises additives conventional in the dosage form in question. Tabletting aids, commonly used in tablet formulation can be used and reference is made to the extensive literature on the subject, see in particular Fiedler's "Lexicon der Hilfstoffe", 4th Edition, ECV Aulendorf 1996. These include but are not limited to disintegrants, binders, lubricants, glidants, stabilising agents, fillers or diluents, surfactants and the like.

Examples of pharmaceutically acceptable disintegrants include, but are not limited to, starches; clays; celluloses; alginates; gums; cross-linked polymers, e.g., cross-linked polyvinyl pyrrolidone or crospovidone, e.g., POLYPLASDONE XL from International Specialty Products (Wayne, NJ); cross-linked sodium carboxymethylcellulose or croscarmellose sodium, e.g., AC-DI-SOL from FMC; and cross-linked calcium carboxymethylcellulose; soy polysaccharides; and guar gum, most preferably cross-linked polyvinyl pyrrolidone or crospovidone. The disintegrant may be present in a concentration from about 0% to about 65%; e.g., from about 1% to about 40%; (e.g., from about 0.05% to about 10%) by weight of the composition (prior to optional coating).

Examples of pharmaceutically acceptable binders include, but are not limited to, starches; celluloses and derivatives thereof, for example, microcrystalline cellulose, e.g., AVICEL PH from FMC (Philadelphia, PA), hydroxypropyl cellulose, in particular low substituted hydroxypropyl cellulose, e.g. hydroxypropyl cellulose having a hydroxypropyl content of 5 to 16 % by weight and a Mw of from 80 000 to 1 150 000, more particularly 140 000 to 850 000, such as LH21, hydroxylethyl cellulose and hydroxylpropylmethyl cellulose METHOCEL from Dow Chemical Corp. (Midland, MI); sucrose; dextrose; corn syrup; polysaccharides; and gelatin, most preferably celluloses such as hydroxypropyl cellulose, in particular low substituted hydroxypropyl cellulose. The binder may be present in a concentration from about 1 to about 60%; e.g., from 5% to about 40% by weight of the composition, in particular from 10% to about 40% by weight of the composition (prior to optional coating), if direct compression methods are employed, or from 5% to about 30% by weight of the composition (prior to optional coating) if roller compaction is employed. Although some of the excipients could also be considered as disintgrants, for the purposes of the present invention they are solely regarded as binders.

Examples of pharmaceutically acceptable fillers and pharmaceutically acceptable diluents include, but are not limited to, confectioner's sugar, compressible sugar, dextrates, dextrin, dextrose, lactose, mannitol, microcrystalline cellulose, in particular Cellulose MK GR, powdered cellulose, sorbitol, and sucrose, in particular microcrystalline cellulose. The filler may be present in a concentration from about 4% to about 60%; e.g. from about 20% to about 40% by weight of the composition (prior to optional coating).

Examples of pharmaceutically acceptable lubricants and pharmaceutically acceptable glidants include, but are not limited to, colloidal silica, magnesium trisilicate, starches, talc, tribasic calcium phosphate, magnesium stearate, aluminum stearate, calcium stearate, magnesium carbonate, magnesium oxide, polyethylene glycol, powdered cellulose, glyceryl behenate, stearic acid, hydrogenated castor oil, glyceryl monostearate, and sodium stearyl fumarate. The glidant may be present in a concentration from 0% to 10%, such as up to 2%, for example approximately 0.1% (prior to optional coating). The lubricant may be present in an amount from 0% to 5%; e.g., from about 0.5% to about 5% (prior to optional coating).

Preferably, it is a characteristic of the present solid oral dosage forms that they contain only a relatively small amount of additives given the high content of active agent. This enables the production of physically small unit dosage forms. The total amount of additives in a given unit dosage may be about 65 % or less by weight based on the total weight of the solid oral dosage form (prior to optional coating), more particularly about 55 % or less. Preferably the additive content is in the range of about 35 to 55 % by weight, more particularly 40 to 45 % by weight.

The absolute amounts of each additive and the amounts relative to other additives is similarly dependent on the desired properties of the solid oral dosage form and may also be chosen by the skilled artisan by routine experimentation without undue burden. For example, the solid oral dosage form may be chosen to exhibit accelerated and/or delayed release of the active agent with or without quantitative control of the release of active agent.

Thus, where accelerated release is desired, e.g. about 90% release within a ten minute, more particularly a five minute period, a disintegrant such as crosslinked polyvinyl pyrrolidone, for example those products known under the registered trade marks Polyplasdone^{®}XL or Kollidon^{®}CL, in particular having a molecular weight in excess of 1 000 000, more particularly having a particle size distribution of less than 400 microns or less than 74 microns, or reactive additives (effervescent mixtures) that effect rapid disintegration of the tablet in the presence of water, for example so-called effervescent tablets that contain an acid in solid form, typically citric acid, which acts in water on a base containing chemically combined carbon dioxide, for example sodium hydrogencarbonate or sodium carbonate, and releases carbon dioxide.

Whereas if delayed release is desired one can employ pellet coating technology, wax matrix systems, polymer matrix tablets or polymer coatings conventional in the art.

Quantitative control of the release of the active agent can be achieved by conventional techniques known in the art. Such dosage forms are known as oral osmotic systems (OROS), coated tablets, matrix tablets, press-coated tablets, multilayer tablets and the like.

In a solid oral dosage form wherein the active agent consist entirely of the dual acting compound trisodium [3-((1S,3R)-1-biphenyl-4-ylmethyl-3-ethoxycarbonyl-1-butylcarbamoyl) propionate-(S)-3'-methyl-2'-(pentanoyl{2"-(tetrazol-5-ylate)biphenyl-4'-ylmethyl}amino)butyrate]hemipentahydrate, preferred additives are microcrystalline cellulose, hydroxypropylcellulose, Crospovidone, Mg, Ca or Al stearate, anhydrous colloidal silica and talc. The amounts of additive employed will depend upon how much active agent is to be used. The stearate, e.g. Mg stearate is preferably employed in amounts of 1.0 to 6.0% by weight, e.g. 1.5% to 4.0 % by weight (prior to optional coating). Whereas the silica is preferably employed in an amount of from 0.1 to 2% by weight. The microcrystalline cellulose is preferably present in an amount of 10 to 30%, e.g. 20-21%. The crosspovidone is preferably present in an amount of 1 to 20 %, more preferably 5 to 15%, e.g. 8-10%.

The solid oral dosage forms according to the present invention may be in the form of dragees in which case the solid oral dosage form is provided with a coating typically a sugar, shellac or other film coating entirely conventional in the art. Attention is drawn to the numerous known methods of coating employed in the art, e.g. spray coating in a fluidized bed, e.g. by the known methods using apparatus available from Aeromatic, Glatt, Wurster or Hüttlin, in a perforated pan by the Accela Cota method, or to the submerged sword coating method. The additives commonly used in confectioning are employed in such methods.

The invention provides in another of its aspects a process of making a solid oral dosage form as hereinabove described. Such solid oral dosage form may be produced by working up the final composition defined hereinabove in appropriate amounts, to form unit dosage forms.

In one embodiment there is provided a process of making the solid oral dosage forms as hereinabove described comprising the steps of
(a) mixing a dual acting compound with at least one pharmaceutically acceptable excipient to form a blend;
(b) directly compressing said blend into a solid oral dosage form.

A further preferred embodiment of the present invention is a process for the manufacture of a solid oral dosage form according to the present invention for higher doses of the dual acting compound. Such a solid oral dosage form can be prepared by the following method, comprising the steps of mixing a dual acting compound with at least one pharmaceutically acceptable excipient to form a blend; compacting, such as roller compacting, said blend; optionally mixing with further pharmaceutically acceptable excipients, and optionally compressing the final blend into a solid oral dosage form.

More particularly, said method comprises the steps of
(a) sieving the dual acting compound and pharmaceutically acceptable excipients to form a sieved material;
(b) blending the sieved material to form a blended material;
(c) compacting, such as roller compacting, the blended material to form a compacted material;
(d) milling the compacted material to form a milled material referred to as the granulate;
(e) optionally blending the milled material with outer phase, i.e., with pharmaceutically acceptable excipients to form a final mixture;
(f) compressing the final blend to form a tablet and
(g) optionally applying a film coat in order to obtain the film coated tablets.

The process is carried out in the absence of water, i.e. it is a dry compression method. The relative humidity is preferably < 55%. The temperature is preferably ambient temperature (20 - 25°C) but can be increased up to 65 °C, such as up to 40 °C. These conditions are preferred to avoid decomposition into the individual components or amorphism of the therapeutic agent.

Compaction to form a coprimate requires the compaction of the dry ground components. Compaction may be carried out using a slugging technique or preferably, roller compaction. Roller compaction apparatus is conventional and essentially utilises two rollers which roll towards each other. A hydraulic ram forces one of the rollers against the other to exert a compacting force against the ground particles fed into the roller compactor via a screw conveyor system. A compaction force of 20 to 60 kN, more preferably 20 to 40 KN, is preferably used. Within this range of compaction forces it has surprisingly been found that the therapeutic agent can be formulated appropriately without decomposition into the individual components or amorphism of the therapeutic agent. The particular optimum compaction force is dependent on the active agent content in any given formulation and therefore also depends on the amount and nature of the additives present.

It was surprising that despite the relatively weak non-covalent forces holding the components of the therapeutic agent together, the above formulation techniques leave the therapeutic agent intact and allow for reliable preparation methods of suitable solid oral dosage forms.

Quantities of ingredients, represented by percentage by weight of the pharmaceutical composition, used in each example are set forth in the respective tables located after the respective descriptions.

### Examples 1 and 2

The therapeutic agent in this example is trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate]hemipentahydrate. Table 1 shows the formulation for Examples 1 and 2 having 5 mg and 50 mg of therapeutic agent respectively.

| **Ingredients** | **Function** | **Example 1 Percentage (w%/w%)** | **Example 2 Percentage (w%/w%)** |
|---|---|---|---|
| **therapeutic agent** | | **4.7** | **9.4** |
| **microcrystalline cellulose** | **filler** | **46.2** | **41.5** |
| **Talc** | **glidant** | **4.3** | **4.3** |
| **low substituted hydroxypropylcellulose** | **binder/disintegrant** | **34.8** | **34.8** |
| **colloidal silicon dioxide** | **glidant** | **0.4** | **0.4** |
| **Crospovidone** | **disintegrant** | **8.7** | **8.7** |
| **magnesium stearate** | **lubricant** | **0.9** | **0.9** |
| **Total** | | **100%** | **100%** |

The therapeutic agent is first sieved through a 40 mesh screen. Added to the therapeutic agent Is microcrystalline cellulose and crospovidone, the mixture is sieved through a 20 mesh screen. The mixture is then blended for about a hundred rotations in a bin blender. The low substituted hydroxypropylcellulose and colloidal silicon dioxide is then added to the bin blender which is then rotated for another hundred rotations. Talc is then added to the mixture and bin blended. The final addition is magnesium stearate. The powdered mixture is then compressed into a tablet weighing about 115 mg for Example 1
and about 575 mg for Example 2. The dissolution profiles of these examples at pH 6.8 are shown in Fig. 1.

### Examples 3 to 6

The therapeutic agent in this example is trisodium [3-((1S,3R)-1-biphenyl-4-ylmethyl-3-ethoxycarbonyl-1-butylcarbamoyl)propionate-(S)-3'-methyl-2'-(pentanoyl{2"-(tetrazol-5-ylate)biphenyl-4'-ylmethyl}amino)butyrate]hemipentahydrate. Tables 2 and 3 show the formulation for Examples 3 to 6 having 40mg, 100 mg, 200 mg and 400 mg of therapeutic agent respectively.

| Ingredients | | | mg/tab |
|---|---|---|---|
| INTRAGRANULAR | | | |
| Therapeutic agent | | | 45.4 |
| Microcrystalline Cellulose | | | 14 |
| L-HPC (low substituted) | | | 10 |
| Crospovidone | | | 4 |
| Colloidal silicon dioxide | | | 0.4 |
| Talc | | | 0.8 |
| Magnesium Stearate | | | 1.4 |

| EXTRA GRANULAR | | | |
|---|---|---|---|
| Crospovidone | | | 3.2 |
| Magnesium Stearate | | | 0.8 |
| Total tablet weight (mg) | | | 80 |

| Ingredients | 100mg | 200mg | 400mg |
|---|---|---|---|
| | mg/Tablet | mg/Tablet | mg/Tablet |
| INTRAGRANULAR | | | |
| LCZ696-ABA.001 | 107.8 | 215.6 | 431.2 |
| Microcrystalline Cellulose (Cellulose MK GR) | 40.2 | 80.4 | 160.8 |
| L-HPC (low sub) | 25.0 | 50.0 | 100.0 |
| Crospovidone | 10.0 | 20.0 | 40.0 |
| Colloidal silicon dioxide | 1.0 | 2.0 | 4.0 |
| Talc | 1.5 | 3.0 | 6.0 |
| Magnesium Stearate | 3.0 | 6.0 | 12.0 |

| EXTRA GRANULAR | | | |
|---|---|---|---|
| Talc | 0.5 | 1.0 | 2.0 |
| Crospovidone | 8.0 | 16.0 | 32.0 |
| Magnesium Stearate | 3.0 | 6.0 | 12.0 |
| Opadry White | 4.43 | 6.63 | 9.95 |
| Opadry Yellow | 2.86 | 4.30 | 6.44 |
| Opadry Red | 0.65 | 0.98 | 1.47 |
| Opadry Black | 0.06 | 0.09 | 0.14 |
| Weight gain per tablet (mg) | 8 | 12 | 18 |
| Total Tablet weight (mg) | 208 | 412 | 818 |

Magnesium stearate, talc, colloidal silicon dioxide and microcrystalline cellulose are first sieved through a 30 mesh screen. The above mixture, the therapeutic agent, crospovidone and low substituted hydroxypropylcellulose are then blended for about 120 rotations in a bin blender. Afterwards, the obtained blend is sieved through a 30 mesh screen. The sieved mixture is then blended for about 120 rotations in a bin blender. The blend is compacted using a roller compactor BEPEX 50 with a compaction force of 30 kN. After compaction, the mixture is milled using a Frewitt Oscillator and sieved through an 18 mesh screen to obtain the final internal phase or granulate. The granulate is blended with crospovidone and talc (external excipients), sieved through a 30 mesh screen, for about 50 rotations in a bin blender. Thereafter, the obtained mixture is blended with magnesium stearate (external excipient), sieved through a 30 mesh screen, for about 50 rotations in a bin blender. The obtained final mixture is then compressed into a tablet using a Fette 3090 apparatus. The coating was applied using Opadry coating polymer to obtain coated tablets. The dissolution profiles of Examples 3 to 5 at pH 6.8 are shown in Fig. 2 and the dissolution profile of Example 6 at pH 4.5 is shown in Fig. 3.

### Example : DISSOLUTION TESTING

The tablets of the Examples are tested for their dissolution in 900 ml of pH 6.8 phosphate buffer with paddles at 50 rpm.
The assembly consists of the following: a covered vessel made of glass or other inert, transparent material; a motor, and a paddle formed from a blade and shaft as the stirring element. The vessel is partially immersed in a suitable water bath of any convenient size or placed in a heating jacket. The water bath or heating jacket permits holding the temperature inside the vessels at 37 ± 0.5° during the test and keeping the bath fluid in constant, smooth motion. No part of the assembly, including the environment in which the assembly is placed, contributes significant motion, agitation, or vibration beyond that due to the smoothly rotating stirring element. Apparatus that permits observation of the specimen and stirring element during the test is has the following dimensions and capacities: the height is 160 mm to 210 mm and its inside diameter is 98 mm to 106 mm. Its sides are flanged at the top. A fitted cover may be used to retard evaporation. The shaft is positioned so that its axis is not more than 2 mm at any point from the vertical axis of the vessel and rotates smoothly without significant wobble. The vertical center line of the blade passes through the axis of the shaft so that the bottom of the blade is flush with the bottom of the shaft. The design of the paddle is as shown in USP <711>, Fig. 2. The distance of 25 ± 2 mm between the blade and the inside bottom of the vessel is maintained during the test. The metallic or suitably inert, rigid blade and shaft comprise a single entity. A suitable two-part detachable design may be used provided the assembly remains firmly engaged during the test. The paddle blade and shaft may be coated with a suitable inert coating. The dosage unit is allowed to sink to the bottom of the vessel before rotation of the blade is started. A small, loose piece of nonreactive material such as not more than a few turns of wire helix may be attached to dosage units that would otherwise float. Other validated sinker devices may be used.
1L of a buffered aqueous solution, adjusted to pH 6.8 ± 0.05 (0.05 M Phosphate buffer solution obtained by dissolving 6.805g of potassium dihydrogen phosphate and 0.896g of sodium hydroxide in and diluting to 1000 ml with water, and adjusting the pH to 6.80±0.05 using 0.2M sodium hydroxide or 1M phosphoric acid; referred hereinafter as "Dissolution Medium") is placed in the vessel of the apparatus, the apparatus is assembled, the Dissolution Medium is equilibrated to 37 ± 0.5°, and the thermometer is removed. 1 dosage form (e.g. tablet or capsule) is placed on the apparatus, taking care to exclude air bubbles from the surface of the dosage-form unit, and immediately the apparatus is operated at a rate of 50+2 rpm. Within the time interval specified (e.g. 10, 20, 30, 45, 60, 90 and 120 min.), or at each of the times stated, a specimen(> 1 ml) is withdrawn from a zone midway between the surface of the Dissolution Medium and the top of the rotating blade, not less than 1 cm from the vessel wall. [NOTE- the aliquots withdrawn for analysis are replaced with equal volumes of fresh Dissolution Mediums at 37° or, where it can be shown that replacement of the medium is not necessary, the volume change is corrected in the calculation. The vessel is kept covered for the duration of the test, and the temperature of the mixture under test at suitable times is verified.] . The specimen is filtered through a suitable filter, e.g. a 0.45 µm PVDF filter (Millipore) and the first mls (2 to 3 ml) of the filtrate are discarded. The analysis is performed by HPLC or UV detection. The test is repeated at least 6 times. with additional dosage form units.

Their dissolution profiles are shown in FIG. 1 and in FIG. 2. Greater than 90% of the therapeutic agent is released from both Example 1 and Example 2 tablets in less than ten minutes and Greater than 70% of the therapeutic agent is released from both Examples 3 to 5 tablets in less than 20 minutes.

The tablets of the Examples can also be tested using the above method at pH 4.5 by carrying out the method as described above and applying the following modifications:
Preparation of pH 4.5 phosphate buffer solution is achieved by dissolving 13.61 g of potassium dihydrogen phosphate in 750 ml of water, adjusting the pH if necessary with 0.1M sodium hydroxide or with 0.1M hydrochoric acid and diluting to 1000.0 ml with water.

### Dissolution testing condition at pH 4.5:

| **Conditions** | |
|---|---|
| Speed of rotation | 75 ± 3 rpm |
| Test medium | Phosphate buffer solution pH 4.5 |
| Volume of test medium | 1000 ml |

The dissolution profile for Example 6 at pH 4.5 is shown in FIG. 3. Greater than 80% of the therapeutic agent is released from Examples 6 tablets in less than 20 minutes.

### Examples : Measurement of pharmacokinetic parameters

### 1) 5 to 80 mg doses:

The study employs a two-period, parallel group, ascending single dose (5, 20, 80 mg of LCZ696 and 40 mg Diovan^{®} (marketed formulation of valsartan), placebo controlled design with dose selection based on FDA exploratory-IND guidance. The dosages of LCZ696 are obtained by using the 5mg and 50mg tablets as manufactured above and employing for the 20mg and 80mg dose multiple tablets of 5mg and/or 50mg strength.

To enable comparison of the exposure to valsartan between LCZ696 and Diovan^{®} at different dose levels, valsartan's exposure (AUC) and Cₘₐₓ values are normalized to the actual amount of anhydrous salt-free- valsartan administered (normalized by mg-equivalent). The AUC₀₋₂₄ values are calculated as ng_{*}h/ml/mg-equivalent of valsartan, and the geometric means are compared. The mean relative bioavailability of valsartan with LCZ696 administration is substantially higher than with Diovan^{®} with the ratio of geometric means for the three LCZ696 cohorts ranging from 107% to 249%. Valsartan exposures following administration of LCZ696 are dose linear and since there is no statistically significant deviations in the dose normalized valsartan exposure between the 3 cohorts, the data from all 3 cohorts is combined (n = 24) to get a pooled estimate of the relative bioavailability of valsartan for LCZ696 compared to 40 mg Diovan^{®}. Exposure (AUC) and Cₘₐₓ values normalized to mg-equivalent of anhydrous salt-free- valsartan are summarized in Table 4.

The rate and extent of absorption of valsartan with LCZ696 is greater than with Diovan^{®}. The dose-equivalent normalized Cₘₐₓ of valsartan is higher following LCZ696 administration than following Diovan^{®} administration (ratio of geometric means for AUC is 161%, 90% CI: 140-185%). Also, a trend towards a shorter tₘₐₓ (mean 1.3-1.8 h) for valsartan is observed following LCZ696 administration than that (mean 2.4-3.0 h) following Diovan^{®} administration.

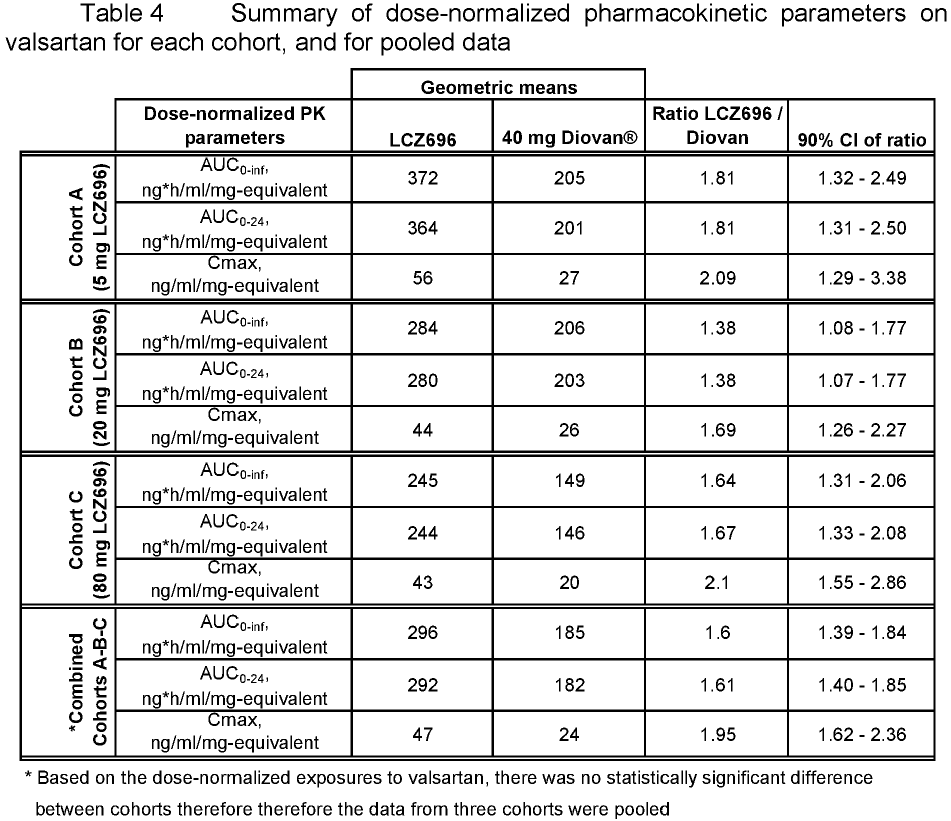

### 2) 50 to 1200 mg doses:

This study uses an interwoven single- and multiple-ascending dose design (randomized, double-blind, placebo controlled, time-lagged, parallel group) to assess safety, tolerability, and pharmacokinetics of LCZ696 in healthy volunteers. The doses for this study are as follows: 200, 600, 900, and 1200 mg for the single dose cohorts; 50, 200, 600, and 900 mg for the multiple dose cohorts lasting 14 days.

Mean tₘₐₓ and t_{1/2} estimates for all analytes are consistent with earlier findings obtained from the above single dose study of lower doses (i.e., Study above).

Minimal accumulation was evident for all analytes following administration of 50-900 mg LCZ696 once daily for 14 days.

**Table 5 Summary of LCZ696 pharmacokinetic parameters for valsartan following single dose administration.**

| | tₘₐₓ (h) | Cₘₐₓ ng/ml | AUC₀₋₂₄ (ng.h/ml) | t_{1/2} (h) |
|---|---|---|---|---|
| 200 mg: | 1.7 | 3309 | 16970 | 11.7 |
| 600 mg : | 1.9 | 7269 | 40645 | 16.6 |
| 900 mg : | 2.2 | 8374 | 53568 | 14.9 |
| 1200 mg: | 2.2 | 7448 | 60118 | 8.9 |

**Table 6 Summary of LCZ696 pharmacokinetic parameters for valsartan following daily administration for 14 days.**

| | tₘₐₓ (h) | Cₘₐₓ ng/ml | AUC₀₋₂₄ (ng.h/ml) | t_{1/2} (h) |
|---|---|---|---|---|
| 50 mg: | 1.6 | 1233 | 6935 | 15.2 |
| 200 mg : | 1.8 | 3990 | 21079 | 22.1 |
| 600 mg : | 2.2 | 8563 | 58876 | 22.6 |
| 900 mg : | 4.9 | 8960 | 54920 | 15.0 |

## Claims

1. A solid oral dosage form in the form of a tablet comprising:
(a) the compound trisodium [3-((1S,3R)-1-biphenyl-4-ylmethyl-3-ethoxycarbonyl-1-butylcarbamoyl) propionate-(S)-3'-methyl-2'-(pentanoyl{2"-(tetrazol-5-ylate)biphenyl-4'-ylmethyl}amino)butyrate]hemipentahydrate in a concentration from about 4% to about 60% by weight of the solid oral dosage form; and
(b) at least one pharmaceutically acceptable excipient.

2. The solid oral dosage form of claim 1, wherein the dose of the compound is 100 mg, 200 mg or 400 mg.

3. The solid oral dosage form of claim 1 or 2, wherein the oral dosage form is an immediate-release formulation.

4. The solid oral dosage form according to any one of claims 1 to 3, wherein the oral dosage form exhibits an *in vitro* dissolution profile, such that, when measured by the USP paddle method at about 50 rpm in 900 mL of 0.05M at pH 6.8 phosphate buffer and at 37±0.5°C, after 10 min, from a mean of about 10% to a mean of about 100 % (by weight) of valsartan free acid, or a pharmaceutically acceptable salt thereof, is released, after 20 min, from a mean of about 30% to a mean of about 100% (by weight) of valsartan free acid, or a pharmaceutically acceptable salt thereof, is released, and after 30 min, from a mean of about 40% to a mean of about 100% (by weight) of valsartan free acid, or a pharmaceutically acceptable salt thereof, is released.

5. The solid oral dosage form according to any one of claims 1 to 3, wherein the oral dosage form provides a rate of absorption of valsartan free acid with a tₘₐₓ of 1 to 2.2 h following administration of a single dose of said dosage form.

6. The solid oral dosage form according to claim 5, wherein the oral dosage form comprises about 200 mg of the compound and provides a rate of absorption of valsartan free acid with a tₘₐₓ of 1.5 to 1.9 h following administration of a single dose of said dosage form.

7. The solid oral dosage form according to claim 4, wherein
when measured by the USP paddle method at about 50 rpm in 900 mL of 0.05M at pH 6.8 phosphate buffer and at 37±0.5°C, or when measured by the USP paddle method at about 75 rpm in 1000 mL of pH 4.5 phosphate buffer and at 37±0.5°C,
(a) when the compound is present in an amount of 100 mg per unit dosage form, the oral dosage form exhibits an *in vitro* dissolution profile such that after 10 min, a mean of 50% of valsartan free acid, is released, after 20 min, a mean of 85% of valsartan free acid, is released, after 30 min, a mean of 95% of valsartan free acid, is released, or
(b) when the compound is present in an amount of 200 mg per unit dosage form, the oral dosage form exhibits an *in vitro* dissolution profile such that after 10 min, a mean of 50% of valsartan free acid, is released, after 20 min, a mean of 85% of valsartan free acid, is released, after 30 min, a mean of 95% of valsartan free acid, is released, or
(c) when the compound is present in an amount of 400 mg per unit dosage form, the oral dosage form exhibits an *in vitro* dissolution profile such that after 10 min, a mean of 40% of valsartan free acid, is released, after 20 min, a mean of 70% of valsartan free acid, is released, after 30 min, a mean of 90% of valsartan free acid, is released.

8. The solid oral dosage form according to any one of claims 1 to 3, wherein the pharmaceutically acceptable excipient is a disintegrant selected from the group consisting of starches, clays, celluloses, alginates, gums, cross-linked polymers, cross-linked sodium carboxymethylcelllose or croscarmellose sodium, cross-linked calcium carboxymethylcellulose, soy polysaccharides, and guar gum, preferably wherein the disintegrant is cross-linked polyvinyl pyrrolidone or crospovidone.

9. The solid oral dosage form according to claim 8, wherein the disintegrant is present in a concentration from 0% to 65% by weight of the composition prior to optional coating, preferably from 1% to 40% by weight of the composition prior to optional coating.

10. The solid oral dosage form according to claim 9, wherein the disintegrant is present in a concentration from 0.05% to 10% by weight of the composition prior to optional coating.

11. The solid oral dosage form according to any one of claims 1 to 3 or claims 8 to 10, wherein the pharmaceutically acceptable excipient is a binder selected from the group consisting of starches, celluloses, sucrose, dextrose, corn syrup, polysaccharides, and gelatin; in particular wherein the binder is hydroxypropyl cellulose.

12. The solid oral dosage form according to claim 11, wherein the binder is hydroxypropyl cellulose.

13. The solid oral dosage form according to claim 12, wherein the binder is hydroxypropyl cellulose having a hydroxypropyl content of 5 to 16% by weight and a MW of from 80 000 to 1 150 000, more particularly 140 000 to 850 000.

14. The solid oral dosage form according to any one of claims 11 to 13, wherein the binder is present in a concentration from 1% to 60% by weight of the composition prior to optional coating, preferably from 5% to 30% by weight of the composition prior to optional coating if the tablet is a roller compacted tablet.

15. The solid oral dosage form according to any one of claims 1 to 3 or claims 8 to 14, wherein the pharmaceutically acceptable excipient is a filler, wherein the filler is selected from the group consisting of confectioner's sugar, compressible sugar, dextrates, dextrin, dextrose, lactose, mannitol, microcrystalline cellulose, powdered cellulose, sorbitol, and sucrose; preferably wherein the filler is microcrystalline cellulose.

16. The solid oral dosage form according to claim 15, wherein the filler is present in a concentration from 4% to 60% by weight of the composition prior to optional coating.

17. The solid oral dosage form according to any one of claims 1 to 3 or claims 8 to 16, wherein the pharmaceutically acceptable excipient is a lubricant or glidant selected from the group consisting of colloidal silica, magnesium trisilicate, starches, tribasic calcium phosphate, magnesium stearate, aluminium stearate calcium stearate, magnesium carbonate, magnesium oxide, polyethylene glycol, powdered cellulose, glyceryl behenate, stearic acid, hydrogenated castor oil, glycerol monostearate, and sodium stearyl fumarate; preferably wherein the lubricant is magnesium stearate.

18. The solid oral dosage form according to claim 17, wherein the glidant is present in a concentration from 0% to 10% by weight of the composition prior to optional coating, preferably up to 2% by weight of the composition prior to optional coating.

19. The solid oral dosage form according to claim 17, wherein the lubricant is present in a concentration from 0% to 5% by weight of the composition prior to optional coating, preferably from 0.5% to 5% by weight of the composition prior to optional coating.

20. The solid oral dosage form according to any one of claims 1-3, 8, 10-13, 15, 17-19, wherein in said solid oral dosage form the total amount of all pharmaceutically acceptable excipients together is in the range of 35% to 55% by weight, more particularly 40% to 45% by weight, of the composition prior to any optional coating.

21. The solid oral dosage form according to any one of claims 1 to 3 or claim 20, wherein the active agent in the solid oral dosage form consists entirely of the compound trisodium [3-((1S,3R)-1-biphenyl-4-ylmethyl-3-ethoxycarbonyl-1- butylcarbamoyl) propionate-(S)-3'-methyl-2'-(pentanoyl{2"-(tetrazol-5- ylate)biphenyl-4'-ylmethyl}amino)butyrate]hemipentahydrate, and the pharmaceutically acceptable excipients comprise (i) microcrystalline cellulose, (ii) hydroxypropylcellulose, (iii) Crospovidone, (iv) Mg, Ca or Al stearate, (v) anhydrous colloidal silica and (vi) talc.

22. The solid oral dosage form according to claim 21, wherein Mg stearate is present in an amount from 1.0 to 6.0% by weight, anhydrous colloidal silica is present in an amount from 0.1 to 2% by weight, microcrystalline cellulose is present in an amount from 10 to 30% by weight, and crospovidone is present in an amount from 1 to 20% by weight, of the composition prior to any optional coating.

23. The solid oral dosage form according to claim 21 or 22, wherein the pharmaceutically acceptable excipient is crospovidone and wherein the crospovidone is present in an amount of 5 to 15% by weight, preferably 8-10% by weight, of the composition prior to any optional coating.

24. The solid oral dosage form according to any preceding claim, wherein the tablet is prepared by a dry formulation process.

25. The solid oral dosage form according to any preceding claim, wherein the tablet is a roller compacted tablet.

26. The solid oral dosage form according to claim 25, wherein the dose of the compound is from 50 to 800 mg.

27. The solid oral dosage form according to claims 25 and 26, wherein the tablet is formed using a compaction force of 20 to 60, preferably 20 to 40 kN.

28. The solid oral dosage form according to any one of claims 25 to 27, wherein the process of manufacture is carried out (i) in the absence of water, and/or (ii) at relative humidity < 55%, and/or (iii) at ambient temperature (20-25°C).

29. The solid oral dosage form according to claim 28, wherein the process of manufacture is carried out (i) in the absence of water, (ii) at relative humidity < 55%, and (iii) at ambient temperature (20-25°C).

30. The solid oral dosage form of any preceding claim for use in therapy, wherein the unit dose of therapeutic agent is 100 mg, 200 mg or 400 mg per day.

## Patentansprüche

1. Feste orale Darreichungsform in Form einer Tablette, umfassend:
(a) die Verbindung Trinatrium-[3-((1S,3R)-1-biphenyl-4-ylmethyl-3-ethoxycarbonyl-1-butylcarbamoyl)propionat-(S)-3'-methyl-2'-(pentanoyl{2"-(tetrazol-5-ylat)biphenyl-4'-ylmethyl}amino)butyrat]-Hemipentahydrat in einer Konzentration von etwa 4 Gew.-% bis etwa 60 Gew.-% der festen oralen Darreichungsform; und
(b) wenigstens einen pharmazeutisch unbedenklichen Exzipienten.

2. Feste orale Darreichungsform nach Anspruch 1, wobei die Dosis der Verbindung bei 100 mg, 200 mg oder 400 mg liegt.

3. Feste orale Darreichungsform nach Anspruch 1 oder 2, wobei es sich bei der oralen Darreichungsform um eine Formulierung mit sofortiger Freisetzung handelt.

4. Feste orale Darreichungsform gemäß einem der Ansprüche 1 bis 3, wobei die orale Darreichungsform ein solches In-vitro-Auflösungsprofil zeigt, dass bei Messung mit der USP-Blatt-Methode bei etwa 50 U/min in 900 ml 0,05 M Phosphatpuffer bei pH 6,8 und 37±0,5°C nach 10 min im Mittel etwa 10 Gew.-% bis im Mittel etwa 100 Gew.-% Valsartan freie Säure bzw. ein pharmazeutisch unbedenkliches Salz davon, nach 20 min im Mittel etwa 30 Gew.-% bis im Mittel etwa 100 Gew.-% Valsartan freie Säure bzw. ein pharmazeutisch unbedenkliches Salz davon und nach 30 min im Mittel etwa 40 Gew.-% bis im Mittel etwa 100 Gew.-% Valsartan freie Säure bzw. ein pharmazeutisch unbedenkliches Salz davon freigesetzt sind.

5. Feste orale Darreichungsform gemäß einem der Ansprüche 1 bis 3, wobei die orale Darreichungsform eine Resorptionsgeschwindigkeit von Valsartan freie Säure mit einer tₘₐₓ von 1 bis 2,2 h nach Verabreichung einer Einzeldosis der Darreichungsform liefert.

6. Feste orale Darreichungsform gemäß Anspruch 5, wobei die orale Darreichungsform etwa 200 mg der Verbindung umfasst und eine Resorptionsgeschwindigkeit von Valsartan freie Säure mit einer tₘₐₓ von 1,5 bis 1,9 h nach Verabreichung einer Einzeldosis der Darreichungsform liefert.

7. Feste orale Darreichungsform gemäß Anspruch 4, wobei bei Messung mit der USP-Blatt-Methode bei etwa 50 U/min in 900 ml 0,05 M Phosphatpuffer bei pH 6,8 und 37±0,5°C oder bei Messung mit der USP-Blatt-Methode bei etwa 75 U/min in 1000 ml Phosphatpuffer bei pH 4,5 und 37±0,5°C,
(a) wenn die Verbindung in einer Menge von 100 mg pro Einheitsdarreichungsform vorliegt, die orale Darreichungsform ein solches In-vitro-Auflösungsprofil zeigt, dass nach 10 min im Mittel 50% Valsartan freie Säure, nach 20 min im Mittel 85% Valsartan freie Säure, nach 30 min im Mittel 95% Valsartan freie Säure freigesetzt sind oder,
(b) wenn die Verbindung in einer Menge von 200 mg pro Einheitsdarreichungsform vorliegt, die orale Darreichungsform ein solches In-vitro-Auflösungsprofil zeigt, dass nach 10 min im Mittel 50% Valsartan freie Säure, nach 20 min im Mittel 85% Valsartan freie Säure, nach 30 min im Mittel 95% Valsartan freie Säure freigesetzt sind oder,
(c) wenn die Verbindung in einer Menge von 400 mg pro Einheitsdarreichungsform vorliegt, die orale Darreichungsform ein solches In-vitro-Auflösungsprofil zeigt, dass nach 10 min im Mittel 40% Valsartan freie Säure, nach 20 min im Mittel 70% Valsartan freie Säure, nach 30 min im Mittel 90% Valsartan freie Säure freigesetzt sind.

8. Feste orale Darreichungsform gemäß einem der Ansprüche 1 bis 3, wobei es sich bei dem pharmazeutisch unbedenklichen Exzipienten um ein Sprengmittel handelt, das aus der Gruppe bestehend aus Stärken, Tonen, Cellulosen, Alginaten, Gummis, vernetzten Polymeren, vernetzter Natriumcarboxymethylcellulose oder Croscarmellose-Natrium, vernetzter Calciumcarboxymethylcellulose, Soja-Polysacchariden und Guarkernmehl ausgewählt ist, bevorzugt wobei es sich bei dem Sprengmittel um vernetztes Polyvinylpyrrolidon oder Crospovidon handelt.

9. Feste orale Darreichungsform gemäß Anspruch 8, wobei das Sprengmittel in einer Konzentration von 0 Gew.-% bis 65 Gew.-% der Zusammensetzung vor optionaler Beschichtung, bevorzugt 1 Gew.-% bis 40 Gew.-% der Zusammensetzung vor optionaler Beschichtung vorliegt.

10. Feste orale Darreichungsform gemäß Anspruch 9, wobei das Sprengmittel in einer Konzentration von 0,05 Gew.-% bis 10 Gew.-% der Zusammensetzung vor optionaler Beschichtung vorliegt.

11. Feste orale Darreichungsform gemäß einem der Ansprüche 1 bis 3 oder der Ansprüche 8 bis 10, wobei es sich bei dem pharmazeutisch unbedenklichen Exzipienten um ein Bindemittel handelt, das aus der Gruppe bestehend aus Stärken, Cellulosen, Saccharose, Dextrose, Glucosesirup, Polysacchariden und Gelatine ausgewählt ist, insbesondere wobei es sich bei dem Bindemittel um Hydroxypropylcellulose handelt.

12. Feste orale Darreichungsform gemäß Anspruch 11, wobei es sich bei dem Bindemittel um Hydroxypropylcellulose handelt.

13. Feste orale Darreichungsform gemäß Anspruch 12, wobei es sich bei dem Bindemittel um Hydroxypropylcellulose mit einem Hydroxypropylgehalt von 5 bis 16 Gew.-% und einem MW von 80 000 bis 1 150 000, ganz besonders 140 000 bis 850 000 handelt.

14. Feste orale Darreichungsform gemäß einem der Ansprüche 11 bis 13, wobei das Bindemittel in einer Konzentration von 1 Gew.-% bis 60 Gew.-% der Zusammensetzung vor optionaler Beschichtung, bevorzugt von 5 Gew.-% bis 30 Gew.-% der Zusammensetzung vor optionaler Beschichtung, falls es sich bei der Tablette um eine walzenkompaktierte Tablette handelt, vorliegt.

15. Feste orale Darreichungsform gemäß einem der Ansprüche 1 bis 3 oder der Ansprüche 8 bis 14, wobei es sich bei dem pharmazeutisch unbedenklichen Exzipienten um einen Füllstoff handelt, wobei der Füllstoff aus der Gruppe bestehend aus Puderzucker, verpressbarem Zucker, Dextraten, Dextrin, Dextrose, Lactose, Mannit, mikrokristalliner Cellulose, Cellulosepulver, Sorbit und Saccharose ausgewählt ist; bevorzugt wobei es sich bei dem Füllstoff um mikrokristalline Cellulose handelt.

16. Feste orale Darreichungsform gemäß Anspruch 15, wobei der Füllstoff in einer Konzentration von 4 Gew.-% bis 60 Gew.-% der Zusammensetzung vor optionaler Beschichtung vorliegt.

17. Feste orale Darreichungsform gemäß einem der Ansprüche 1 bis 3 oder der Ansprüche 8 bis 16, wobei es sich bei dem pharmazeutisch unbedenklichen Exzipienten um ein Gleitmittel oder Fließmittel handelt, das aus der Gruppe bestehend aus Kieselsol, Magnesiumtrisilicat, Stärken, Calciumphosphat, Magnesiumstearat, Aluminiumstearat, Calciumstearat, Magnesiumcarbonat, Magnesiumoxid, Polyethylenglykol, Cellulosepulver, Glycerylbehenat, Stearinsäure, hydriertem Ricinusöl, Glycerylmonostearat und Natriumstearylfumarat ausgewählt ist; bevorzugt wobei es sich bei dem Gleitmittel um Magnesiumstearat handelt.

18. Feste orale Darreichungsform gemäß Anspruch 17, wobei das Fließmittel in einer Konzentration von 0 Gew.-% bis 10 Gew.% der Zusammensetzung vor optionaler Beschichtung, bevorzugt bis zu 2 Gew.-% der Zusammensetzung vor optionaler Beschichtung vorliegt.

19. Feste orale Darreichungsform gemäß Anspruch 17, wobei das Gleitmittel in einer Konzentration von 0 Gew.-% bis 5 Gew.% der Zusammensetzung vor optionaler Beschichtung, bevorzugt von 0,5 Gew.-% bis 5 Gew.% der Zusammensetzung vor optionaler Beschichtung vorliegt.

20. Feste orale Darreichungsform gemäß einem der Ansprüche 1-3, 8, 10-13, 15, 17-19, wobei in der festen oralen Darreichungsform die Gesamtmenge aller pharmazeutisch unbedenklichen Exzipienten zusammen im Bereich von 35 Gew.-% bis 55 Gew.-%, ganz besonders 40 Gew.-% bis 45 Gew.-%, der Zusammensetzung jeweils vor optionaler Beschichtung liegt.

21. Feste orale Darreichungsform gemäß einem der Ansprüche 1 bis 3 oder Anspruch 20, wobei der Wirkstoff in der festen oralen Darreichungsform vollständig aus der Verbindung Trinatrium-[3-((1S,3R)-1-biphenyl-4-ylmethyl-3-ethoxycarbonyl-1-butylcarbamoyl)propionat-(S)-3'-methyl-2'-(pentanoyl{2"-(tetrazol-5-ylat)-biphenyl-4'-ylmethyl}amino)butyrat]-Hemipentahydrat besteht und die pharmazeutisch unbedenklichen Exzipienten (i) mikrokristalline Cellulose, (ii) Hydroxypropylcellulose, (iii) Crospovidon, (iv) Magnesium-, Calcium- oder Aluminiumstearat, (v) wasserfreies Kieselsol und (vi) Talkum umfassen.

22. Feste orale Darreichungsform gemäß Anspruch 21, wobei Magnesiumstearat in einer Menge von 1,0 bis 6,0 Gew.-%, wasserfreies Kieselsol in einer Menge von 0,1 bis 2 Gew.-%, mikrokristalline Cellulose in einer Menge von 10 bis 30 Gew.-% und Crospovidon in einer Menge von 1 bis 20 Gew.-% der Zusammensetzung jeweils vor optionaler Beschichtung vorliegen.

23. Feste orale Darreichungsform gemäß Anspruch 21 oder 22, wobei es sich bei dem pharmazeutisch unbedenklichen Exzipienten um Crospovidon handelt und wobei das Crospovidon in einer Menge von 5 bis 15 Gew.-%, bevorzugt 8-10 Gew.-%, der Zusammensetzung jeweils vor optionaler Beschichtung vorliegt.

24. Feste orale Darreichungsform gemäß einem vorhergehenden Anspruch, wobei die Tablette mit einem Trockenformulierungsverfahren hergestellt wird.

25. Feste orale Darreichungsform gemäß einem vorhergehenden Anspruch, wobei es sich bei der Tablette um eine walzenkompaktierte Tablette handelt.

26. Feste orale Darreichungsform gemäß Anspruch 25, wobei die Dosis der Verbindung bei 50 bis 800 mg liegt.

27. Feste orale Darreichungsform gemäß Anspruch 25 und 26, wobei die Tablette unter Verwendung einer Kompaktierkraft von 20 bis 60 kN, bevorzugt 20 bis 40 kN, geformt wird.

28. Feste orale Darreichungsform gemäß einem der Ansprüche 25 bis 27, wobei das Herstellungsverfahren (i) in Abwesenheit von Wasser und/oder (ii) bei < 55% relative Luftfeuchtigkeit und/oder (iii) bei Umgebungstemperatur (20-25°C) durchgeführt wird.

29. Feste orale Darreichungsform gemäß Anspruch 28, wobei das Herstellungsverfahren (i) in Abwesenheit von Wasser, (ii) bei < 55% relative Luftfeuchtigkeit und (iii) bei Umgebungstemperatur (20-25°C) durchgeführt wird.

30. Feste orale Darreichungsform nach einem vorhergehenden Anspruch zur therapeutischen Verwendung, wobei die Einheitsdosis an Therapeutikum bei 100 mg, 200 mg oder 400 mg pro Tag liegt.

## Revendications

1. Forme pharmaceutique orale solide sous la forme d'un comprimé comprenant :
(a) le composé [3-((1S,3R)-1-biphényl-4-ylméthyl-3-éthoxycarbonyl-1-butylcarbamoyl)propionate-(S)-3'-méthyl-2'-(pentanoyl{2"-(tétrazol-5-ylate)biphényl-4'-ylméthyl}amino)butyrate]hémipentahydrate trisodique à une concentration d'environ 4 % à environ 60 % en poids de la forme pharmaceutique orale solide ; et
(b) au moins un excipient pharmaceutiquement acceptable.

2. Forme pharmaceutique orale solide selon la revendication 1, dans laquelle la dose du composé est de 100 mg, 200 mg ou 400 mg.

3. Forme pharmaceutique orale solide selon la revendication 1 ou 2, la forme pharmaceutique orale étant une formulation à libération immédiate.

4. Forme pharmaceutique orale solide selon l'une quelconque des revendications 1 à 3, la forme pharmaceutique orale présentant un profil de dissolution *in vitro*, de sorte que, lorsqu'il est mesuré par le procédé à palettes USP à environ 50 tours/min dans 900 ml de tampon phosphate 0,05 M à pH 6,8 et à 37 ± 0,5 °C, après 10 min, une moyenne d'environ 10 % à une moyenne d'environ 100 % (en poids) de valsartan acide libre, ou un sel pharmaceutiquement acceptable de celui-ci, est libérée, après 20 min, une moyenne d'environ 30 % à une moyenne d'environ 100 % (en poids) de valsartan acide libre, ou un sel pharmaceutiquement acceptable de celui-ci, est libérée, et après 30 min, une moyenne d'environ 40 % à une moyenne d'environ 100 % (en poids) de valsartan acide libre, ou un sel pharmaceutiquement acceptable de celui-ci, est libérée.

5. Forme pharmaceutique orale solide selon l'une quelconque des revendications 1 à 3, la forme pharmaceutique orale présentant un taux d'absorption de valsartan acide libre avec un tₘₐₓ de 1 à 2,2 h après administration d'une dose unique de ladite forme pharmaceutique.

6. Forme pharmaceutique orale solide selon la revendication 5, la forme pharmaceutique orale comprenant environ 200 mg du composé et présentant un taux d'absorption de valsartan acide libre avec un tₘₐₓ de 1,5 à 1,9 h après administration d'une dose unique de ladite forme pharmaceutique.

7. Forme pharmaceutique orale solide selon la revendication 4, dans laquelle
en mesurant par le procédé à palettes USP à environ 50 tours/min dans 900 ml de tampon phosphate 0,05 M à pH 6,8 et à 37 ± 0,5 °C, ou en mesurant par le procédé à palettes USP à environ 75 tours/min dans 1000 ml de tampon phosphate pH 4,5 et à 37 ± 0,5 °C,
(a) lorsque le composé est présent en une quantité de 100 mg par forme pharmaceutique unitaire, la forme pharmaceutique orale présente un profil de dissolution *in vitro* tel qu'après 10 min, une moyenne de 50 % de valsartan acide libre est libérée, après 20 min, une moyenne de 85 % de valsartan acide libre est libérée, après 30 min, une moyenne de 95 % de valsartan acide libre est libérée, ou
(b) lorsque le composé est présent en une quantité de 200 mg par forme pharmaceutique unitaire, la forme pharmaceutique orale présente un profil de dissolution *in vitro* tel qu'après 10 min, une moyenne de 50 % de valsartan acide libre est libérée, après 20 min, une moyenne de 85 % de valsartan acide libre est libérée, après 30 min, une moyenne de 95 % de valsartan acide libre est libérée, ou
(c) lorsque le composé est présent en une quantité de 400 mg par forme pharmaceutique unitaire, la forme pharmaceutique orale présente un profil de dissolution *in vitro* tel qu'après 10 min, une moyenne de 40 % de valsartan acide libre est libérée, après 20 min, une moyenne de 70 % de valsartan acide libre est libérée, après 30 min, une moyenne de 90 % de valsartan acide libre est libérée.

8. Forme pharmaceutique orale solide selon l'une quelconque des revendications 1 à 3, dans laquelle l'excipient pharmaceutiquement acceptable est un délitant choisi dans le groupe constitué des suivants : amidons, argiles, celluloses, alginates, gommes, polymères réticulés, carboxyméthylcellulose sodique ou croscarmellose sodique réticulée, carboxyméthylcellulose calcique réticulée, polysaccharides de soja, et gomme de guar, de préférence dans laquelle le délitant est la polyvinylpyrrolidone ou crospovidone réticulée.

9. Forme pharmaceutique orale solide selon la revendication 8, dans laquelle le délitant est présent à une concentration de 0 % à 65 % en poids de la composition avant enrobage facultatif, de préférence de 1 % à 40 % en poids de la composition avant enrobage facultatif.

10. Forme pharmaceutique orale solide selon la revendication 9, dans laquelle le délitant est présent à une concentration de 0,05 % à 10 % en poids de la composition avant enrobage facultatif.

11. Forme pharmaceutique orale solide selon l'une quelconque des revendications 1 à 3 ou des revendications 8 à 10, dans laquelle l'excipient pharmaceutiquement acceptable est un liant choisi dans le groupe constitué des suivants : amidons, celluloses, saccharose, dextrose, sirop de maïs, polysaccharides et gélatine ; en particulier dans laquelle le liant est l'hydroxypropylcellulose.

12. Forme pharmaceutique orale solide selon la revendication 11, dans laquelle le liant est l'hydroxypropylcellulose.

13. Forme pharmaceutique orale solide selon la revendication 12, dans laquelle le liant est l'hydroxypropylcellulose ayant une teneur en hydroxypropyle de 5 à 16 % en poids et un poids moléculaire de 80 000 à 1 150 000, plus particulièrement 140 000 à 850 000.

14. Forme pharmaceutique orale solide selon l'une quelconque des revendications 11 à 13, dans laquelle le liant est présent à une concentration de 1 % à 60 % en poids de la composition avant enrobage facultatif, de préférence de 5 % à 30 % en poids de la composition avant enrobage facultatif si le comprimé est un comprimé compacté au rouleau.

15. Forme pharmaceutique orale solide selon l'une quelconque des revendications 1 à 3 ou des revendications 8 à 14, dans laquelle l'excipient pharmaceutiquement acceptable est une charge, dans laquelle la charge est choisie dans le groupe constitué des suivants : sucre glace, sucre compressible, dextrates, dextrine, dextrose, lactose, mannitol, cellulose microcristalline, cellulose pulvérulente, sorbitol et saccharose ; de préférence dans laquelle la charge est la cellulose microcristalline.

16. Forme pharmaceutique orale solide selon la revendication 15, dans laquelle la charge est présente à une concentration de 4 % à 60 % en poids de la composition avant enrobage facultatif quelconque.

17. Forme pharmaceutique orale solide selon l'une quelconque des revendications 1 à 3 ou des revendications 8 à 16, dans laquelle l'excipient pharmaceutiquement acceptable est un lubrifiant ou un agent glissant choisi dans le groupe constitué des suivants : silice colloïdale, trisilicate de magnésium, amidons, phosphate de calcium tribasique, stéarate de magnésium, stéarate d'aluminium, stéarate de calcium, carbonate de magnésium, oxyde de magnésium, polyéthylène glycol, cellulose pulvérulente, béhénate de glycéryle, acide stéarique, huile de ricin hydrogénée, monostéarate de glycérol et fumarate de stéaryle de sodium ; de préférence dans laquelle le lubrifiant est le stéarate de magnésium.

18. Forme pharmaceutique orale solide selon la revendication 17, dans laquelle l'agent glissant est présent à une concentration de 0 % à 10 % en poids de la composition avant enrobage facultatif, de préférence jusqu'à 2 % en poids de la composition avant enrobage facultatif.

19. Forme pharmaceutique orale solide selon la revendication 17, dans laquelle le lubrifiant est présent à une concentration de 0 % à 5 % en poids de la composition avant enrobage facultatif, de préférence de 0,5 % à 5 % en poids de la composition avant enrobage facultatif.

20. Forme pharmaceutique orale solide selon l'une quelconque des revendications 1 à 3, 8, 10 à 13, 15, 17 à 19, dans laquelle, dans ladite forme pharmaceutique orale solide, la quantité totale de l'ensemble des excipients pharmaceutiquement acceptables est dans la plage de 35 % à 55 % en poids, plus particulièrement 40 % à 45 % en poids, de la composition avant un enrobage facultatif.

21. Forme pharmaceutique orale solide selon l'une quelconque des revendications 1 à 3 ou la revendication 20, dans laquelle l'agent actif dans la forme pharmaceutique orale solide est constitué entièrement du composé [3-((1S,3R)-1-biphényl-4-ylméthyl-3-éthoxycarbonyl-1-butylcarbamoyl)propionate-(S)-3'-méthyl-2'-(pentanoyl{2"-(tétrazol-5-ylate)biphényl-4'-ylméthyl}amino)butyrate]hémipentahydrate trisodique, et les excipients pharmaceutiquement acceptables comprennent (i) la cellulose microcristalline, (ii) l'hydroxypropylcellulose, (iii) la crospovidone, (iv) le stéarate de Mg, Ca ou Al, (v) la silice colloïdale anhydre et (vi) le talc.

22. Forme pharmaceutique orale solide selon la revendication 21, dans laquelle le stéarate de Mg est présent en une quantité de 1,0 à 6,0 % en poids, la silice colloïdale anhydre est présente en une quantité de 0,1 à 2 % en poids, la cellulose microcristalline est présente en une quantité de 10 à 30 % en poids, et la crospovidone est présente en une quantité de 1 à 20 % en poids, de la composition avant un enrobage facultatif quelconque.

23. Forme pharmaceutique orale solide selon la revendication 21 ou 22, dans laquelle l'excipient pharmaceutiquement acceptable est la crospovidone et dans laquelle la crospovidone est présente en une quantité de 5 à 15 % en poids, de préférence 8 à 10 % en poids, de la composition avant un enrobage facultatif quelconque.

24. Forme pharmaceutique orale solide selon l'une quelconque des revendications précédentes, dans laquelle le comprimé est préparé par un procédé de formulation sèche.

25. Forme pharmaceutique orale solide selon l'une quelconque des revendications précédentes, dans laquelle le comprimé est un comprimé compacté au rouleau.

26. Forme pharmaceutique orale solide selon la revendication 25, dans laquelle la dose du composé est de 50 à 800 mg.

27. Forme pharmaceutique orale solide selon les revendications 25 et 26, dans laquelle le comprimé est formé en utilisant une force de compactage de 20 à 60, de préférence 20 à 40 kN.

28. Forme pharmaceutique orale solide selon l'une quelconque des revendications 25 à 27, le procédé de fabrication étant conduit (i) en l'absence d'eau, et/ou (ii) à une humidité relative < 55 %, et/ou (iii) à température ambiante (20 à 25 °C).

29. Forme pharmaceutique orale solide selon la revendication 28, le procédé de fabrication étant conduit (i) en l'absence d'eau, (ii) à une humidité relative < 55 %, (iii) à température ambiante (20 à 25 °C).

30. Forme pharmaceutique orale solide selon l'une quelconque des revendications précédentes pour utilisation en thérapie, dans laquelle la dose unitaire d'agent thérapeutique est 100 mg, 200 mg ou 400 mg par jour.
